# EUROPEAN PATENT APPLICATION

(11) **EP 4 417 043 A1**
(43) Date of publication of application: **21.08.2024**
(21) Application number: 22910545.7
(22) Date of filing: 19.10.2022
(51) Int. Cl.: A01K 23/00

(54) **DIAPER FOR PETS**

(30) Priority: 20.12.2021 JP 2021206043
(71) Applicant: Unicharm Corporation, Shikokuchuo-city, Ehime 799-0111 (JP)
(72) Inventor: KOMATSUBARA, Daisuke, Kanonji-shi, Kagawa 769-1602 (JP); KOIDO, Yumi, Kanonji-shi, Kagawa 769-1602 (JP); FUKUMOTO, Daichi, Kanonji-shi, Kagawa 769-1602 (JP)
(74) Representative: Dolleymores
(86) International application number: PCT/JP2022/038965
(87) International publication number: WO 2023/119822

(57) **Abstract**

A pet diaper according to one aspect includes: an absorbent body having a width direction disposed along a waistline direction and a longitudinal direction connecting a ventral side and a dorsal side of the pet, the absorbent body including an absorbent core having absorbency; and a pair of fastening tabs extending outward from both edges of the absorbent body in the width direction. A tail hole through which a tail of the pet passes is formed in the absorbent body. The absorbent body includes a center portion in which the absorbent core is disposed and a pair of side portions protruding outward from edges of the absorbent core in the width direction. A maximum width of the absorbent core in the width direction is larger than a maximum width of each of the pair of side portions in the width direction, and a width in the width direction of at least a portion of a region of the absorbent core located between the pair of fastening tabs is narrowed toward the tail hole.

## Description

### Technical Field

The present disclosure relates to a pet diaper.

### Background Art

Pet diapers worn on pets such as dogs and cats have been known. For example, Patent Literatures 1 and 2 below disclose pet diapers each including a diaper body including an absorbent core, a pair of fastening tabs extending outward from both side peripheries of the diaper body in the width direction, and a target portion engaged with the pair of fastening tabs. The diaper body includes a center portion in which the absorbent core is disposed and a pair of side portions protruding from side edges of the absorbent core to both sides in the width direction. A tail hole through which a tail of a pet passes is formed at a position between the absorbent core of the diaper body and the target portion.

### Citation List

### Patent Literature

Patent Literature 1: Japanese Unexamined Patent Publication No. 2017-85906
Patent Literature 2: Japanese Patent No. 4490080

### Summary of Invention

### Technical Problem

In recent years, there is an increasing demand for a diaper for small animals such as puppies, small dogs, and cats. In this type of diaper for small animals, a width of a side portion is generally formed to be small since waistlines of small animals are short. Here, in a case where the width of the side portion is formed to be smaller than a width of an absorbent core, the absorbent core protrudes laterally, and thus, legs of a pet sometimes come into contact with the absorbent core during walking. When the legs of the pet come into contact with the absorbent core, the pet diaper is sometimes displaced to induce urine leakage. On the other hand, when the width of the absorbent core is made smaller in order to avoid interference between the legs of the pet and the absorbent core, urine absorption performance is deteriorated, and urine leakage is likely to occur.

Therefore, an object of the present disclosure is to provide a pet diaper capable of suppressing urine leakage.

### Solution to Problem

A pet diaper according to one aspect includes: an absorbent body having a width direction disposed along a waistline direction and a longitudinal direction connecting a ventral side and a dorsal side of the pet, the absorbent body including an absorbent core having absorbency; and a pair of fastening tabs extending outward from both edges of the absorbent body in the width direction. A tail hole through which a tail of the pet passes is formed in the absorbent body. The absorbent body includes a center portion in which the absorbent core is disposed and a pair of side portions protruding outward from edges of the absorbent core in the width direction. A maximum width of the absorbent core in the width direction is larger than a maximum width of each of the pair of side portions in the width direction, and a width in the width direction of at least a portion of a region of the absorbent core located between the pair of fastening tabs is narrowed toward the tail hole.

In the pet diaper according to the present aspect, since the maximum width of the absorbent core in the width direction is formed to be larger than the maximum width of each of the pair of side portions in the width direction, urine can be sufficiently absorbed in the absorbent core. Furthermore, since the width in the width direction of at least the portion of the region located between the pair of fastening tabs of the absorbent core is narrowed toward the tail hole, legs of the pet hardly come into contact with the absorbent core during walking. Therefore, displacement of the pet diaper is less likely to occur, and urine leakage can be suppressed.

In one embodiment, a maximum width of the absorbent body in the width direction may be larger than half of a maximum length of the absorbent body in the longitudinal direction. Since a small animal such as a puppy often has a body shape with a bulged ventral part, fitting properties of the pet diaper with respect to the small animal can be improved by relatively increasing the maximum width of the absorbent body in the width direction.

In one embodiment, the absorbent core may include a ventral-side end portion disposed on the ventral side, a dorsal-side end portion disposed on the dorsal side, and a constricted portion disposed between the ventral-side end portion and the dorsal-side end portion and having a width smaller than widths of the ventral-side end portion and the dorsal-side end portion in the width direction, and a length of the constricted portion in the longitudinal direction may be 80% or more and 90% or less of a total length of the absorbent core in the longitudinal direction. In the present embodiment, since the length of the constricted portion, which has a relatively small width, accounts for 80% or more and 90% or less of the total length of the absorbent core, the absorbent core hardly interferes with the legs of the pet during walking. Meanwhile, since the widths of the ventral-side end portion and the dorsal-side end portion of the absorbent core are relatively large, urine leakage from the end portions of the absorbent core in the longitudinal direction can be suppressed.

In one embodiment, a boundary between the dorsal-side end portion and the constricted portion may be located on the dorsal side with respect to a center line of the absorbent body in the longitudinal direction. Since the boundary between the dorsal-side end portion and the constricted portion is disposed on the dorsal side with respect to the center line of the absorbent body in the longitudinal direction, the constricted portion is disposed at a crotch of the pet, and the pet diaper can be easily worn.

In one embodiment, a pair of elastic members fixed to the absorbent body in a state of being stretched in the longitudinal direction and extending in the longitudinal direction may be further provided, a width of the region of the absorbent core in the width direction may be larger than a distance between the pair of elastic members in the width direction, and a minimum width of the absorbent core may be equal to the distance between the pair of elastic members in the width direction or larger than the distance between the pair of elastic members. Since the width of the region located between the pair of fastening tabs is made larger, urine leakage from the ventral-side end portion of the absorbent core can be suppressed. Meanwhile, since the minimum width of the absorbent core is made smaller, the pet diaper is easily worn on the pet.

In one embodiment, when regions obtained by dividing the absorbent core into three equal parts in the longitudinal direction are defined as a dorsal-side region, an intermediate region, and a ventral-side region, respectively, in order of proximity to the tail hole, the area of the dorsal-side region as viewed from a skin surface side of the pet may be larger than the area of the ventral-side region as viewed from the skin surface side. Since urine can be effectively absorbed in the dorsal-side region by increasing the area of the dorsal-side region, urine leakage from the tail hole is less likely to occur.

In one embodiment, the area of the intermediate region as viewed from the skin surface side may be smaller than the area of the dorsal-side region and the area of the ventral-side region. Since the area of the intermediate region is made smaller, the pet diaper is easily worn on the pet.

In one embodiment, a width of the absorbent core in the width direction may be minimum in the dorsal-side region. Since the dorsal-side region of the absorbent core is disposed near the crotch of the pet, when the width of the absorbent core is minimized in the dorsal-side region, the pet diaper is easily worn on the pet.

In one embodiment, the width of the absorbent core in the width direction may be minimum at a position closer to the tail hole than a center line of the absorbent core in the longitudinal direction. Since the width of the absorbent core is minimum at the position closer to the tail hole than the center line of the absorbent core in the longitudinal direction, a portion having the minimum width of the absorbent core is disposed near the crotch of the pet, and thus, the pet diaper is easily worn on the pet.

In one embodiment, a target portion disposed closer to the dorsal side than the pair of fastening tabs and configured to be engaged with the pair of fastening tabs may be further provided, and a width of the pair of fastening tabs in the longitudinal direction may be 3/4 or less of a width of the target portion in the longitudinal direction. Incidentally, the width of the pair of fastening tabs in the longitudinal direction may be 1/2 or less of the width of the target portion in the longitudinal direction. Since the width of the pair of fastening tabs is set to 3/4 or less or 1/2 or less of the width of the target portion, the pair of fastening tabs can be coupled to the target portion in a state of overlapping each other in the waistline direction of the pet. As a result, a length of the waistline of the pet diaper can be shortened, and thus, the pet diaper can be fitted to a small animal having a small waistline.

In one embodiment, an edge on the dorsal side of the absorbent core may be disposed closer to the ventral side than the tail hole. Since the edge on the dorsal side of the absorbent core is disposed closer to the ventral side than the tail hole, regions where the absorbent core is not disposed are formed on both sides in the width direction and the dorsal side of the tail hole. These regions have low rigidity, and thus, are flexibly deformed along the rump of the pet. Therefore, the fitting properties of the pet diaper are improved, a gap is less likely to occur between the edge of the tail hole and the rump of the pet, and urine leakage from the gap can be suppressed.

### Advantageous Effects of Invention

According to various aspects and embodiments of the present invention, the urine leakage can be suppressed.

### Brief Description of Drawings

FIG. 1 is a plan view illustrating a pet diaper according to one embodiment.
FIG. 2 is a cross-sectional view taken along line A-A of FIG. 1.
FIG. 3 is a view illustrating a state in which the pet diaper is worn.
FIG. 4 is a plan view of an absorbent core.
FIG. 5 is a view illustrating an example of a method for fastening a fastening tab.

### Description of Embodiments

Hereinafter, embodiments of the present disclosure will be described with reference to the drawings. Incidentally, in the following description, the same or equivalent elements will be denoted by the same reference signs, and redundant descriptions thereof will not be repeated. Dimensional ratios of the drawings do not always coincide with those of the description.

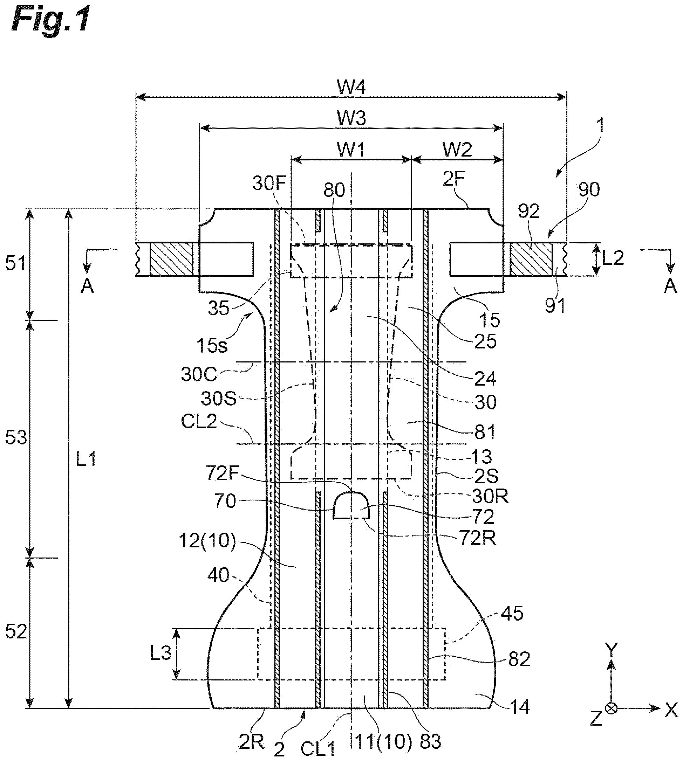

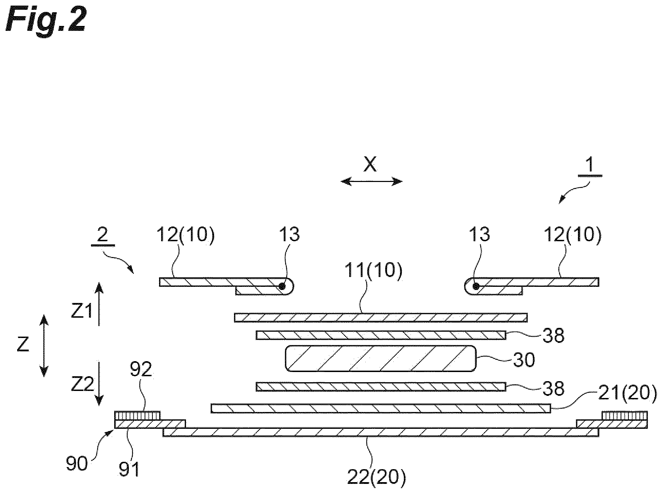

FIG. 1 is a plan view of a pet diaper according to an embodiment as viewed from a skin surface side Z1. FIG. 2 is a cross-sectional view schematically illustrating the pet diaper taken along line A-A illustrated in FIG. 1. FIG. 1 illustrates a pet diaper 1 in a stretched state in which the pet diaper 1 is stretched to a state in which no wrinkle is formed. In the following description, the positional relationship in the stretched state will be described unless otherwise specified. Further, members are illustrated to be spaced apart in a thickness direction Z in the cross-sectional view illustrated in FIG. 2 for convenience of the description, but are in contact with each other in the thickness direction Z in an actual product.

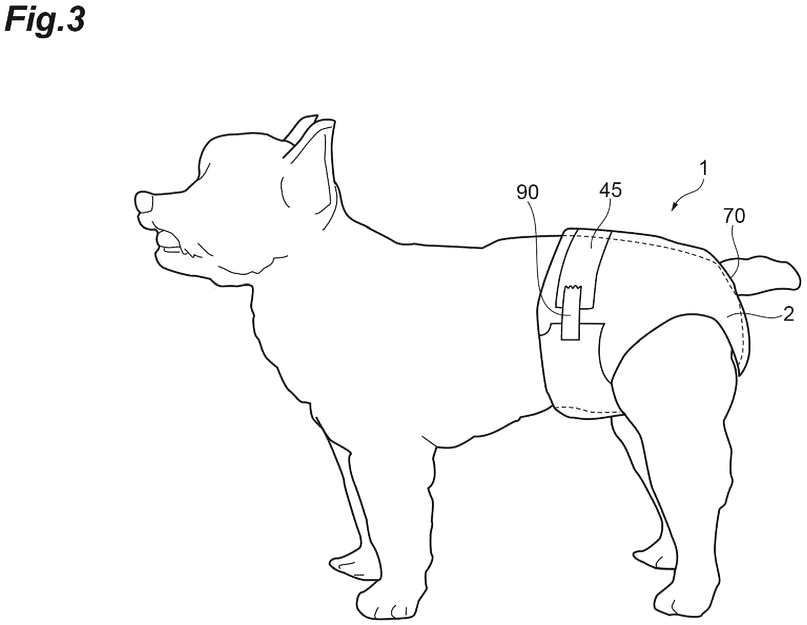

The pet diaper 1 is a diaper used for a pet. In the present specification, the term "pet" broadly encompasses vertebrates and invertebrates, and typically includes pets such as dogs, cats, rabbits, and hamsters. The pet diaper 1 of one embodiment is particularly suitably used as a diaper for a small animal such as a puppy, a small dog, and a cat. As illustrated in FIG. 1, the pet diaper 1 extends in a width direction X disposed along a waistline direction of the pet and in a longitudinal direction Y orthogonal to the width direction X. The longitudinal direction Y is a direction connecting a ventral side to a dorsal side of the pet. The thickness direction Z is a direction orthogonal to the width direction X and the longitudinal direction Y, and includes a skin surface side Z1 facing a skin surface of the pet in a worn state and a non-skin surface side Z2 facing a side opposite to the skin surface of the pet in the worn state. As illustrated in FIG. 3, the pet diaper 1 is worn so as to cover from the ventral side to the dorsal side through a crotch of the pet.

The pet diaper 1 includes an absorbent body 2. The absorbent body 2 absorbs urine excreted by the pet wearing the pet diaper 1. The absorbent body 2 includes a top sheet 10, a back sheet 20, and an absorbent core 30. The top sheet 10 forms a surface of the absorbent body 2 that abuts on the pet, and is disposed on the skin surface side Z1. The top sheet 10 has liquid permeability that allows the urine of the pet to permeate toward the absorbent core 30. The top sheet 10 includes a center sheet 11 that is located at the center in the width direction X and covers the absorbent core 30 and side sheets 12 covering both side portions of the center sheet 11 in the width direction X.

As illustrated in FIG. 2, inner portions of the side sheets 12 in the width direction X are folded back to the non-skin surface side Z2. An elastic member 13 in a state of being stretched in the longitudinal direction Y is disposed between the folded side sheets 12. The side sheets 12 and the elastic member 13 constitute a leakproof gather 80. The leakproof gather 80 is an orthostatic leakproof gather and is disposed closer to the skin surface side Z1 than the absorbent core 30. Details of the leakproof gather 80 will be described later.

The back sheet 20 forms a surface of the absorbent body 2 located outside when being worn, and is disposed on the non-skin surface side Z2. The back sheet 20 includes a liquid impermeable back film 21 and a back nonwoven fabric 22 located closer to the non-skin surface side Z2 than the back film 21. A length of the back film 21 in the width direction X may be formed to be shorter than a length of the back nonwoven fabric 22 in the width direction X, and the back nonwoven fabric 22 may extend to both sides in the width direction X from the back film 21.

The absorbent core 30 is disposed between the top sheet 10 and the back sheet 20. The absorbent core 30 contains, for example, a plant-derived pulp fiber having absorbency and a superabsorbent polymer (SAP), and absorbs the urine of the pet. As illustrated in FIG. 2, an upper surface and a lower surface of the absorbent core 30 may be covered by core wraps 38. The absorbent core 30 is disposed substantially at the center of the absorbent body 2 in the width direction X. A maximum width W1 of the absorbent core 30 in the width direction X is shorter than a maximum width W3 of the absorbent body 2 in the width direction X.

An edge 30R on the dorsal side of the absorbent core 30 is disposed on the dorsal side with respect to a center line CL2 of the absorbent body 2 in the longitudinal direction Y. An edge 30F on the ventral side of the absorbent core 30 is disposed closer to the dorsal side than a ventral-side edge 2F to be described later. That is, a length of the absorbent core 30 in the longitudinal direction Y is shorter than a total length L1 of the absorbent body 2 in the longitudinal direction Y. Incidentally, the center line CL2 is an imaginary line that is located equidistantly from a dorsal-side edge 2R to be described later and the ventral-side edge 2F of the absorbent body 2 and extends along the width direction X. That is, the center line CL2 is a portion where a fold line is formed when the pet diaper 1 is folded in half in the longitudinal direction Y When the pet diaper 1 is worn on the pet, a urination opening of the pet abuts on a region on the ventral side with respect to the center line CL2 of the absorbent body 2.

The absorbent body 2 has the ventral-side edge 2F located on the ventral side in the longitudinal direction Y and the dorsal-side edge 2R located on the dorsal side in the longitudinal direction Y The ventral-side edge 2F is disposed in the waistline direction of the pet on the ventral side when being worn, and the dorsal-side edge 2R is disposed in the waistline direction of the pet on the dorsal side when being worn. A distance between the ventral-side edge 2F and the dorsal-side edge 2R in the longitudinal direction Y is the total length (maximum length) L1 of the absorbent body 2. In one embodiment, a maximum width W3 of the absorbent body 2 in the width direction X may be larger than half of the total length L1 of the absorbent body 2 in the longitudinal direction.

Further, the absorbent body 2 includes a ventral-side area 51 located on the ventral-side edge 2F side, a dorsal-side area 52 located on the dorsal-side edge 2R side, and an intermediate area 53 located between the ventral-side area 51 and the dorsal-side area 52 in the longitudinal direction Y. In the dorsal-side area 52 of the absorbent body 2, a pair of flap portions 14 protruding outward in the width direction X from the absorbent body 2 is formed. In the ventral-side area 51 of the absorbent body 2, a pair of flap portions 15 protruding outward in the width direction X from the absorbent body 2 is formed. The pair of flap portions 15 define a pair of leg openings 15s through which hind legs of the pet pass. A width between side edges of the pair of flap portions 15 in the width direction X corresponds to the maximum width W3 of the absorbent body 2.

The pair of flap portions 15 is provided with a pair of fastening tabs 90, respectively. The pair of fastening tabs 90 has a rectangular shape that is long in the width direction X when viewed from the skin surface side Z1, and is disposed adjacently to the ventral-side edge 2F of the absorbent body 2 in the longitudinal direction Y. The pair of fastening tabs extends outward from side edges 2S of the absorbent body 2 in the width direction X. The pair of fastening tabs 90 includes base material sheets 91 joined to the flap portions 15 and joint portions 92 provided on the base material sheets 91, respectively. The joint portion 92 is disposed on a surface on the skin surface side Z1 of the fastening tab 90. The joint portion 92 is, for example, a mechanical fastener, and is configured to be joinable to a target portion 45 formed on the non-skin surface side Z2 of the absorbent body 2. In one embodiment, the edge 30F on the ventral side of the absorbent core 30 may be located between the pair of flap portions 15.

The target portion 45 is provided on the non-skin surface side Z2 of the dorsal-side area 52 of the absorbent body 2. The target portion 45 is disposed closer to the dorsal-side edge 2R than the pair of fastening tabs 90, and is engaged with the pair of fastening tabs 90. Incidentally, the absorbent body 2 may be configured such that the target portion 45 is not provided and the joint portions 92 of the pair of fastening tabs 90 are directly joined to the back nonwoven fabric 22 of the back sheet 20.

As illustrated in FIG. 1, the target portion 45 has a belt-like shape extending in the width direction X. In one embodiment, a width L2 of the pair of fastening tabs 90 in the longitudinal direction Y is smaller than a width L3 of the target portion 45 in the longitudinal direction Y. For example, the width L2 of the pair of fastening tabs 90 may be 3/4 or less or 1/2 or less of the width L3 of the target portion 45. Incidentally, the width L2 of the pair of fastening tabs 90 and the width L3 of the target portion 45 mean a maximum width of the pair of fastening tabs 90 in the longitudinal direction Y and a maximum width of the target portion 45 in the longitudinal direction Y, respectively.

A tail hole 70 is formed in the absorbent body 2. The tail hole 70 is, for example, a substantially tongue-shaped opening formed in the absorbent body 2 by making a substantially U-shaped cut that is open to the dorsal side. As illustrated in FIG. 3, a tail of the pet passes through the tail hole 70. As illustrated in FIG. 1, the tail hole 70 is disposed between the center line CL2 of the absorbent body 2 in the longitudinal direction Y and the target portion 45 in the longitudinal direction Y. More specifically, the tail hole 70 is disposed closer to the dorsal side than the edge 30R on the dorsal side of the absorbent core 30. Therefore, a region where the absorbent core 30 is not disposed is formed outside the tail hole 70 in the width direction X and on the dorsal side of the tail hole 70.

The tail hole 70 is formed on a center line CL1 of the absorbent body 2 in the width direction X. Incidentally, the center line CL1 is an imaginary line that is located equidistantly from the pair of side edges 2S of the absorbent body 2 in the width direction X and extends along the longitudinal direction Y. Incidentally, the shape of the tail hole 70 is not limited to the substantially tongue shape, and may be a semicircular shape, a rectangular shape, a trapezoidal shape, a polygonal shape, or an elliptical shape. Incidentally, the tail hole 70 may be a substantially tongue-shaped opening formed in the absorbent body 2 by making a substantially U-shaped cut that is open to the ventral side.

A tongue piece 72 is formed in the absorbent body 2. The tongue piece 72 is, for example, a flap-shaped member formed by making a cut 50 in the absorbent body 2, and is configured by, for example, a part of the top sheet 10 and a part of the back sheet 20. The tongue piece 72 is disposed so as to overlap the tail hole 70 when not folded back. The tongue piece 72 has a base end portion 72R connected to an edge on the dorsal side of the tail hole 70 and a distal end portion 72F located on a side opposite to the base end portion 72R. The base end portion 72R of the tongue piece 72 is connected to the intermediate area 53 of the absorbent body 2. On the other hand, the distal end portion 72F is not connected to the intermediate area 53 of the absorbent body 2 and is free. The distal end portion 72F of the tongue piece 72 faces the ventral side in a state where the tongue piece 72 is not folded back (overlapping the tail hole 70).

The tongue piece 72 is configured to open the tail hole 70 by being folded back to the non-skin surface side Z2 with the base end portion 72R as a starting point. As described above, since the distal end portion 72F of the tongue piece 72 faces the ventral side in the embodiment illustrated in FIG. 1, when the tail is inserted into the tail hole 70 and the tongue piece 72 is folded back to the non-skin surface side Z2 with the base end portion 72R as the starting point, the tongue piece 72 abuts on the back side of the tail.

The leakproof gather 80 is formed on the side sheets 12. The leakproof gather 80 is disposed outside the absorbent core 30 in the width direction X, and prevents the urine excreted to the absorbent core 30 from leaking in the width direction X. In one embodiment, the leakproof gather 80 includes a pair of the elastic members 13 extending in the longitudinal direction Y, standing portions 81 that stand upright due to contraction of the pair of elastic members 13, lateral fixed portions 82 serving as standing fulcrums of the standing portions 81 in the width direction X, and vertical fixed portions 83 serving as standing fulcrums of the standing portions 81 in the longitudinal direction Y.

The pair of elastic members 13 is provided in the inner portions of the side sheets 12 in the width direction X and extends in the longitudinal direction Y. The pair of elastic members 13 is made of, for example, a stretchable material such as rubber or spandex that can contract in the length direction. The pair of elastic members 13 is disposed to be spaced apart from each other with the center line CL1 of the absorbent body 2 in the width direction X therebetween, and is fixed to the side sheets 12 in the state of being stretched in the longitudinal direction Y. For example, end portions on the ventral side of the pair of elastic members 13 are disposed in the ventral-side area 51, and end portions on the dorsal side of the pair of elastic members 13 are disposed in the intermediate area 53.

The lateral fixed portions 82 is formed in substantially the entire area of the absorbent body 2 in the longitudinal direction Y at a position outside the standing portion 81 in the width direction X on the side sheet 12. That is, the lateral fixed portions 82 are disposed outside the absorbent core 30 in the width direction X. The lateral fixed portion 82 is a part of the side sheet 12, and is fixed to the center sheet 11. The lateral fixed portions 82 serve as the standing fulcrums of the standing portions 81 in the width direction X.

The vertical fixed portions 83 are formed on each of the ventral side and the dorsal side of the absorbent body 2 at positions inside in the width direction X with respect to the lateral fixed portions 82. The vertical fixed portion 83 is a part of the side sheet 12 and is fixed to the center sheet 11. The vertical fixed portions 83 serve as the standing fulcrums of the standing portions 81 in the longitudinal direction Y.

The standing portions 81 are formed between the vertical fixed portions 83 formed on each of the ventral side and the dorsal side of the absorbent body 2. That is, the standing portions 81 are disposed at positions inside in the width direction X with respect to the lateral fixed portions 82 and at positions inside in the longitudinal direction Y with respect to the vertical fixed portions 83. The standing portion 81 is a portion of each of the side sheets 12 that is not fixed to the center sheet 11. The elastic members 13 stretched in the longitudinal direction Y are connected to the standing portions 81. When the elastic members 13 contract in the longitudinal direction Y, the absorbent body 2 is deformed in a closing direction, and the standing portions 81 rise from the lateral fixed portions 82 as starting points. As the standing portions 81 stand upright at position of sandwiching the absorbent core 30 from the width direction X in this manner, the urine leakage is prevented.

The pet diaper 1 may further include a pair of elastic members 40 stretchable in the length direction. As illustrated in FIG. 1, the pair of elastic members 40 is disposed outside the elastic members 13 in the width direction X. In the embodiment illustrated in FIG. 1, end portions on the ventral side of the pair of elastic members 40 are disposed in the ventral-side area 51, and end portions on the dorsal side of the pair of elastic members 40 are disposed in the dorsal-side area 52. The pair of elastic members 40 is made of a stretchable material such as rubber or spandex that can contract in the length direction, for example, and is disposed along the side edge 2S of the absorbent body 2 between the top sheet 10 and the back sheet 20 of the absorbent body 2. Incidentally, the pair of elastic members 40 may extend in the longitudinal direction Y in a state of being sewn to the top sheet 10 and the back sheet 20 of the absorbent body 2. When the pet diaper 1 is worn, the pair of elastic members 40 contracts in the longitudinal direction Y, whereby the side edges 2S of the absorbent body 2 fit around legs of a wearer.

As illustrated in FIG. 1, the absorbent body 2 includes a center portion 24 in which the absorbent core 30 is disposed, and a pair of side portions 25 protruding outward in the width direction X from side edges 30S of the absorbent core 30. The center portion 24 is a region overlapping the absorbent core 30 in the thickness direction Z of the absorbent body 2. The pair of side portions 25 is regions of the absorbent body 2 including the pair of flap portions 15 each of which is disposed between the side edge 30S of the absorbent core 30 in the width direction X and the side edge 2S of the absorbent body 2. A maximum width of the center portion 24 in the width direction X coincides with the maximum width W1 of the absorbent core 30 in the X direction. The maximum width W1 of the absorbent core 30 in the width direction X is larger than a maximum width W2 of each of the pair of side portions 25 in the width direction X. The maximum width W2 of each of the pair of side portions 25 in the width direction X corresponds to, for example, a distance in the width direction X between the edge 30F on the ventral side of the absorbent core 30 and the side edge of the flap portion 15.

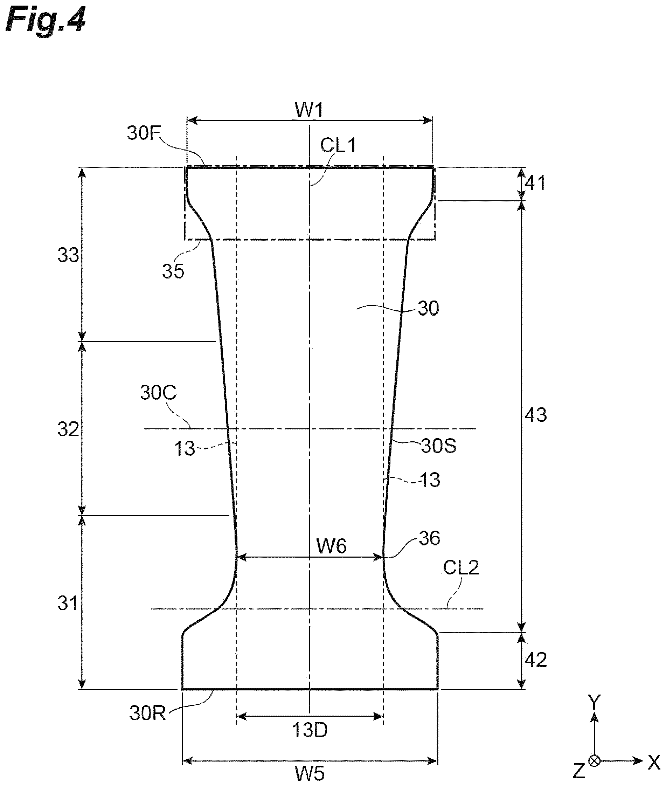

Hereinafter, the absorbent core 30 will be described in more detail. FIG. 4 is a plan view of the absorbent core 30 according to one embodiment. As illustrated in FIG. 4, the edge 30F on the ventral side of the absorbent core 30 has the width W1 in the width direction X. The edge 30F on the ventral side of the absorbent core 30 is a portion having the maximum width of the absorbent core 30. The edge 30R on the dorsal side of the absorbent core 30 has a width W5 in the width direction X. The width W5 of the edge 30R on the dorsal side of the absorbent core 30 may be the same width as the width W1 of the edge 30F on the ventral side of the absorbent core 30. The width of the edge 30F on the ventral side of the absorbent core 30, that is, the maximum width W1 of the absorbent core 30 in the width direction X is larger than a distance 13D between the pair of elastic members 13 in the width direction X.

Further, as illustrated in FIG. 4, the absorbent core 30 includes a ventral-side end portion 41 disposed on the ventral side, a dorsal-side end portion 42 disposed on the dorsal side, and a constricted portion 43 disposed between the ventral-side end portion 41 and the dorsal-side end portion 42. The ventral-side end portion 41 is a region including the edge 30F on the ventral side of the absorbent core 30, and has the width W1 in the width direction X. The dorsal-side end portion 42 is a region including the edge 30R on the dorsal side of the absorbent core 30, and has the width W5 in the width direction X. That is, the ventral-side end portion 41 and the dorsal-side end portion 42 have a constant width in the width direction X. The constricted portion 43 is a region in which the width of the absorbent core 30 is narrowed. A width of the constricted portion 43 in the width direction X is smaller than the width W1 of the ventral-side end portion 41 and the width W5 of the dorsal-side end portion 42 in the entire area of the constricted portion 43.

The constricted portion 43 includes a narrowed portion 36 having the narrowest width in the width direction X. A width W6 of the narrowed portion 36 is smaller than the width W1 of the ventral-side end portion 41 and the width W5 of the dorsal-side end portion 42. The width of the constricted portion 43 is narrowed continuously or stepwise from a boundary between the ventral-side end portion 41 and the constricted portion 43 toward the narrowed portion 36. That is, it can be said that the boundary between the ventral-side end portion 41 and the constricted portion 43 is a starting point of constriction of the absorbent core 30. Similarly, the width of the constricted portion 43 is narrowed continuously or stepwise from a boundary between the dorsal-side end portion 42 and the constricted portion 43 toward the narrowed portion 36. That is, it can be said that the boundary between the dorsal-side end portion 42 and the constricted portion 43 is a starting point of constriction of the absorbent core 30. In one embodiment, a minimum width of the absorbent core 30 in the width direction X, that is, the width W6 of the narrowed portion 36 may be larger than the distance 13D between the pair of elastic members 13.

The narrowed portion 36 is disposed on the ventral side (the edge 30F side) with respect to the center line CL2 of the absorbent body 2 in the longitudinal direction Y, and is disposed on the dorsal side (the tail hole 70 side) with respect to a center line 30C of the absorbent core 30 in the longitudinal direction Y. The center line 30C is an imaginary line that is located equidistantly from the edge 30F on the ventral side and the edge 30R on the dorsal side of the absorbent core 30 and extends along the width direction X. Therefore, in the longitudinal direction Y, the narrowed portion 36 is formed at a position closer to the boundary between the dorsal-side end portion 42 and the constricted portion 43 than the boundary between the ventral-side end portion 41 and the constricted portion 43. Since the narrowed portion 36 is formed at the above-described position, the narrowed portion 36 can be disposed near the crotch of the pet. Therefore, the pet diaper 1 can be easily attached and detached.

As illustrated in FIG. 4, the boundary between the ventral-side end portion 41 and the constricted portion 43 is located on the ventral side with respect to the center line CL2 of the absorbent body 2 in the longitudinal direction Y. On the other hand, the boundary between the dorsal-side end portion 42 and the constricted portion 43 is located on the dorsal side with respect to the center line CL2 of the absorbent body 2 in the longitudinal direction Y. A length of the constricted portion 43 in the longitudinal direction Y is formed to be longer than lengths of the ventral-side end portion 41 and the dorsal-side end portion 42 in the longitudinal direction Y. Incidentally, the length of the ventral-side end portion 41 represents a distance in the longitudinal direction Y between the edge 30F on the ventral side of the absorbent core 30 and the boundary between the ventral-side end portion 41 and the constricted portion 43, and the length of the dorsal-side end portion 42 represents a distance in the longitudinal direction Y between the edge 30R on the dorsal side of the absorbent core 30 and the boundary between the dorsal-side end portion 42 and the constricted portion 43. Further, the length of the constricted portion 43 in the longitudinal direction Y represents a distance in the longitudinal direction Y between the boundary between the ventral-side end portion 41 and the constricted portion 43 and the boundary between the dorsal-side end portion 42 and the constricted portion 43. For example, the length of the constricted portion 43 may be 80% or more and 90% or less with respect to the total length (that is, the distance in the longitudinal direction Y between the edge 30F on the ventral side and the edge 30R on the dorsal side of the absorbent core 30) of the absorbent core 30 in the longitudinal direction Y.

Further, the length of the dorsal-side end portion 42 in the longitudinal direction Y may be longer than the length of the ventral-side end portion 41 in the longitudinal direction Y. As a result, as illustrated in FIG. 4, when regions obtained by dividing the absorbent core 30 into three equal parts in the longitudinal direction Y are defined as a dorsal-side region 31, an intermediate region 32, and a ventral-side region 33, respectively, in order of proximity to the tail hole 70, the area of the dorsal-side region 31 as viewed from the skin surface side Z1 may be larger than the area of the ventral-side region 33 as viewed from the skin surface side Z1. When the area of the dorsal-side region 31 is made larger, the urine can be effectively absorbed in the dorsal-side region 31, and the urine leakage from the tail hole 70 is suppressed. Further, the area of the intermediate region 32 as viewed from the skin surface side Z1 may be smaller than the area of the dorsal-side region 31 and the area of the ventral-side region 33 as viewed from the skin surface side Z1. When the area of the intermediate region 32 is made smaller, the pet diaper 1 can be easily attached and detached.

Incidentally, the narrowed portion 36 of the absorbent core 30 may be disposed in the dorsal-side region 31. That is, the width of the absorbent core 30 in the width direction X is minimum in the dorsal-side region 31.

As illustrated in FIGS. 1 and 4, the absorbent core 30 includes a region (hereinafter, referred to as a "tab region 35") located between the pair of fastening tabs 90. The tab region 35 is disposed so as to straddle the boundary between the ventral-side end portion 41 and the constricted portion 43. Therefore, a part on the ventral side of the tab region 35 has a constant width W1, and a part on the dorsal side of the tab region 35 has a width smaller than the width W1. That is, the width of a portion of the tab region 35 on the dorsal side in the width direction X is narrowed toward the tail hole 70 (the dorsal side).

FIG. 3 is a view illustrating a state in which the pet diaper 1 is worn on the pet. When the pet diaper 1 is worn on the pet, the tail of the pet is first caused to pass through the tail hole 70 to be taken out to the non-skin surface side Z2 of the diaper. At this time, the tongue piece 72 is folded back to the non-skin surface side Z2, and the tongue piece 72 is disposed on the back side of the tail. Next, the ventral-side edge 2F of the absorbent body 2 is caused to abut on the belly of the pet. Then, the dorsal-side edge 2R of the absorbent body 2 is caused to abut on the dorsal part of the pet while causing the vicinity of the center line CL2 of the absorbent body 2 in the longitudinal direction Y to abut on the urination opening of the pet. Next, the pair of fastening tabs 90 is pulled toward the back of the pet in the waistline direction, and the pair of fastening tabs 90 is fastened to an outer surface of the target portion 45 of the absorbent body 2. As a result, the pet diaper 1 is worn so as to cover the belly, the back, and the crotch of the pet as illustrated in FIG. 3.

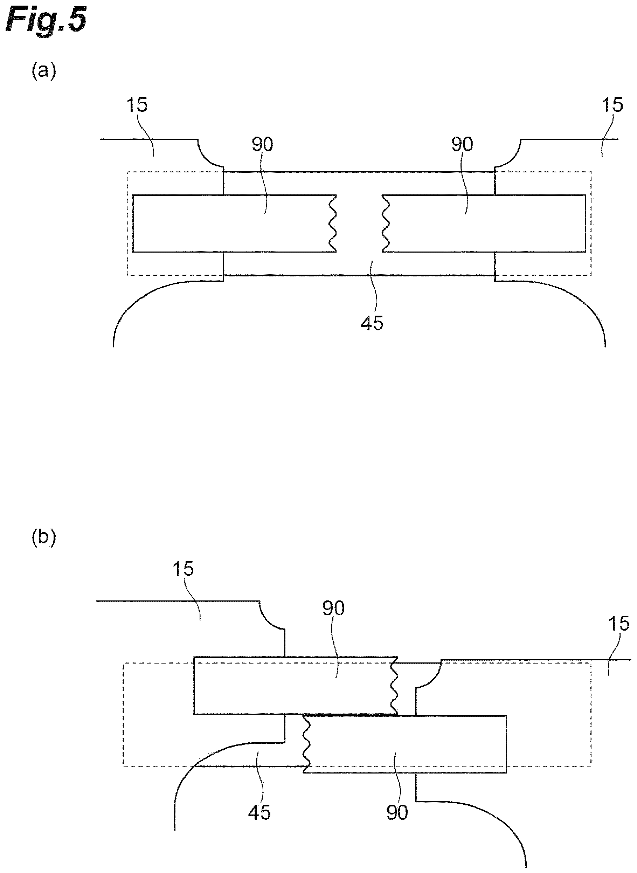

At this time, in a case where the waistline of the pet is larger than a width W4 (see FIG. 1) between end portions of the pair of fastening tabs 90, the pair of fastening tabs is coupled to the target portion 45 so as not to overlap each other in the waistline direction of the pet as illustrated in FIG. 5(a). On the other hand, in a case where the waistline of the pet is smaller than the width W4 between the end portions of the pair of fastening tabs 90, the pair of fastening tabs 90 is coupled to the target portion 45 in a state of overlapping each other in the waistline direction of the pet as illustrated in FIG. 5(b). Since a length of the waistline of the pet diaper is shortened by disposing the pair of fastening tabs 90 to overlap in the waistline direction of the pet, the pet diaper can be fitted even to a small animal having a very small waistline.

In a case where the width W2 of the pair of side portions 25 of the pet diaper 1 is smaller than the maximum width W1 of the absorbent core 30, the absorbent core 30 sometimes protrude laterally when the pet diaper 1 is worn on the pet, and the legs of the pet may come into contact with the absorbent core 30 during walking. In this regard, the width of a portion of the tab region 35 on the dorsal side in the width direction X is narrowed as approaching the tail hole 70 in the above-described pet diaper 1, and thus, the legs of the pet hardly come into contact with the absorbent core 30. On the other hand, when the pair of fastening tabs 90 is joined to the target portion 45, the ventral-side end portion 41 of the absorbent core 30 is in close contact with the ventral part of the pet, and thus, hardly comes into contact with the legs of the pet. Since the width of the ventral-side end portion 41 is the maximum width W1 of the absorbent body 2 in the pet diaper 1, it is possible to effectively absorb the urine of the pet at the ventral-side end portion 41 while suppressing the absorbent core 30 from interfering with the legs of the pet. Therefore, the urine leakage from the pet diaper 1 can be suppressed.

Further, since the width L2 of the pair of fastening tabs 90 in the longitudinal direction Y is 3/4 or less of the width L3 of the target portion in the longitudinal direction Y in the pet diaper 1, most or the whole of the pair of fastening tabs 90 can be engaged with the target portion 45 even when the pair of fastening tabs 90 is fastened to the target portion 45 in a state of overlapping each other in the waistline direction of the pet as illustrated in FIG. 5(b). Therefore, the pet diaper 1 can be fitted to a small animal while preventing the pair of fastening tabs 90 from being detached from the target portion 45. As a result, it is possible to suppress the occurrence of displacement of the pet diaper 1, particularly, rear displacement in which the ventral-side edge 2F of the absorbent body 2 moves to the crotch side of the pet.

Further, since the width of the tab region 35 of the absorbent core 30 is larger than the distance 13D between the pair of elastic members 13 in the width direction X as illustrated in FIG. 4, the standing portions 81 of the leakproof gather 80 easily stand upright due to the rigidity of the tab region 35. Furthermore, since the minimum width W6 of the absorbent core is equal to or less than the distance 13D between the pair of elastic members 13, the pet diaper 1 can be easily attached to a small animal having a short distance between hind legs.

Although the pet diapers according to various embodiments have been described above, the present invention is not limited to the above-described embodiments, and various modifications can be made within a scope not changing the gist of the invention. That is, it should be noted that the above-described embodiments are for the purpose of illustration and are not intended to limit the scope of the present invention. The various embodiments described above can be combined within a scope having no inconsistency.

For example, the width of a part of the tab region 35 in the width direction X is narrowed as approaching the tail hole 70 in the embodiment illustrated in FIG. 4, but in one embodiment, the width of the tab region 35 may be narrowed continuously or stepwise as approaching the tail hole 70 in the entire area of the tab region 35 in the longitudinal direction. Further, the edge 30F on the ventral side of the absorbent core 30 may be disposed on the ventral side of the pair of flap portions 15.

### Reference Signs List

- 1: pet diaper
- 13: elastic member
- 24: center portion
- 25: side portion
- 30: absorbent core
- 31: dorsal-side region
- 32: intermediate region
- 33: ventral-side region
- 41: ventral-side end portion
- 42: dorsal-side end portion
- 43: constricted portion
- 45: target portion
- 70: tail hole
- 90: fastening tab

## Claims

1. A pet diaper comprising:
an absorbent body having a width direction disposed along a waistline direction and a longitudinal direction connecting a ventral side and a dorsal side of a pet, the absorbent body including an absorbent core having absorbency; and
a pair of fastening tabs extending outward from both edges of the absorbent body in the width direction,
wherein a tail hole through which a tail of the pet passes is formed in the absorbent body,
the absorbent body includes a center portion in which the absorbent core is disposed and a pair of side portions protruding outward from edges of the absorbent core in the width direction,
a maximum width of the absorbent core in the width direction is larger than a maximum width of each of the pair of side portions in the width direction, and
a width in the width direction of at least a portion of a region of the absorbent core located between the pair of fastening tabs is narrowed toward the tail hole.

2. The pet diaper according to claim 1, wherein
a maximum width of the absorbent body in the width direction is larger than half of a maximum length of the absorbent body in the longitudinal direction.

3. The pet diaper according to claim 1 or 2, wherein
the absorbent core includes a ventral-side end portion disposed on the ventral side, a dorsal-side end portion disposed on the dorsal side, and a constricted portion disposed between the ventral-side end portion and the dorsal-side end portion and having a width smaller than widths of the ventral-side end portion and the dorsal-side end portion in the width direction, and
a length of the constricted portion in the longitudinal direction is 80% or more and 90% or less of a total length of the absorbent core in the longitudinal direction.

4. The pet diaper according to claim 3, wherein
a boundary between the dorsal-side end portion and the constricted portion is located on the dorsal side with respect to a center line of the absorbent body in the longitudinal direction.

5. The pet diaper according to any one of claims 1 to 4, further comprising
a pair of elastic members fixed to the absorbent body in a state of being stretched in the longitudinal direction and extending in the longitudinal direction,
wherein a width of the region of the absorbent core in the width direction is larger than a distance between the pair of elastic members in the width direction, and
a minimum width of the absorbent core is equal to the distance between the pair of elastic members in the width direction or larger than the distance between the pair of elastic members.

6. The pet diaper according to any one of claims 1 to 5, wherein
when regions obtained by dividing the absorbent core into three equal parts in the longitudinal direction are defined as a dorsal-side region, an intermediate region, and a ventral-side region, respectively, in order of proximity to the tail hole, an area of the dorsal-side region as viewed from a skin surface side of the pet is larger than an area of the ventral-side region as viewed from the skin surface side.

7. The pet diaper according to claim 6, wherein
an area of the intermediate region as viewed from the skin surface side is smaller than the area of the dorsal-side region and the area of the ventral-side region.

8. The pet diaper according to claim 6 or 7, wherein
a width of the absorbent core in the width direction is minimum in the dorsal-side region.

9. The pet diaper according to any one of claims 1 to 8, wherein
a width of the absorbent core in the width direction is minimum at a position closer to the tail hole than a center line of the absorbent core in the longitudinal direction.

10. The pet diaper according to any one of claims 1 to 9, further comprising
a target portion disposed closer to the dorsal side than the pair of fastening tabs and configured to be engaged with the pair of fastening tabs,
wherein a width of the pair of fastening tabs in the longitudinal direction is 3/4 or less of a width of the target portion in the longitudinal direction.

11. The pet diaper according to any one of claims 1 to 9, further comprising
a target portion disposed closer to the dorsal side than the pair of fastening tabs and configured to be engaged with the pair of fastening tabs,
wherein a width of the pair of fastening tabs in the longitudinal direction is 1/2 or less of a width of the target portion in the longitudinal direction.

12. The pet diaper according to any one of claims 1 to 11, wherein
an edge on the dorsal side of the absorbent core is disposed closer to the ventral side than the tail hole.
